Europäisches Patentamt

European Patent Office

Office européen des brevets

(11) Numéro de publication: **0 124 443**
**A2**

## (12) DEMANDE DE BREVET EUROPEEN

(21) Numéro de dépôt: **84400853.2**

(51) Int. Cl.³: **A 61 F 1/03**

(22) Date de dépôt: **26.04.84**

(30) Priorité: **02.05.83 FR 8307250**

(43) Date de publication de la demande: **07.11.84**
**Bulletin 84/45**

(84) Etats contractants désignés: **BE CH DE GB IT LI LU NL**

(71) Demandeur: **COMMISSARIAT A L'ENERGIE ATOMIQUE Etablissement de Caractère Scientifique Technique et Industriel, 31/33, rue de la Fédération, F-75015 Paris (FR)**

(72) Inventeur: **Gabard, Jean-Jacques, 17 Route des Alluets Le Bois Beulle Bazemont, F-78580 Maule (FR)**

(74) Mandataire: **Mongrédien, André et al, c/o BREVATOME 25, rue de Ponthieu, F-75008 Paris (FR)**

(54) **Prothèse fémorale de la hanche.**

(57) Prothèse fémorale de la hanche composée d'une queue (1) et d'une tête (4), caractérisée en ce que la queue se termine par une pièce mâle ou fût (3) d'une longueur de l'ordre de 3 cm ayant une section en forme d'arbre cannelé, tandis que la boule ou la tête fémorale (4) présente une cavité cylindrique (5) de même section se prolongeant en (6) hors de la sphère de façon à former dans son ensemble une pièce femelle ayant une longueur au moins égale à celle de la pièce mâle ci-dessus.

EP 0 124 443 A2

00124443

## PROTHESE FEMORALE DE LA HANCHE

Lors de lésions dégénératives aiguës ou chroniques de la hanche ou lors de fracture du col fémoral ou du cotyle, il est quelquefois nécessaire de remplacer les parties atteintes ou fracturées par des implants prothétiques, au niveau du fémur et au niveau du bassin.

Dans certains cas, tels que des fractures du col du fémur sans lésion dégénérative du bassin, il est suffisant d'effectuer un implant prothétique fémoral qui vient s'insérer dans le cotyle du bassin. Cependant, il arrive que se produise plus tard une lésion dégénérative du cotyle et il devient alors nécessaire de remplacer la prothèse implantée par une prothèse totale.

Dans de tels cas, il est souhaité par les chirurgiens de n'avoir pas à extraire la queue de la prothèse fémorale en place, mais de pouvoir seulement en changer la tête et la remplacer par une tête de plus petit diamètre, correspondant à la cupule de la prothèse cotyloïdienne qu'il y a lieu de mettre en place.

Dans ce but, plusieurs solutions ont déjà été proposées.

L'une d'elles consiste à utiliser des boules ou sphères fémorales interchangeables montées par vissage sur un boulon porté par la queue. Cette proposition s'est traduite par de nombreux échecs dus au fait que les mouvements pendulaires de la hanche provoquant peu à peu un dévissage de la boule, ce qui entraîne des accidents et la nécessité de nouvelles interventions.

Une autre consiste à prévoir des boules fémorales à ouverture conique emmanchées à force sur un cône terminal porté par la queue. Cette solution,

B 8275.3 AM

connue sous le nom d'emboîtage à cône morse, ne donne pas non plus de bons résultats, car cet emmanchement devient trop solide après quelque temps de service, équivalent à une soudure, et les efforts d'extraction qu'il nécessite entraînent des accidents de descellement de la queue ou de brisure de l'os adjacent.

La présente invention a pour objet une prothèse fémorale de la hanche qui remédie à ces inconvénients.

La prothèse selon l'invention se compose d'une queue diaphysaire sur laquelle est montée une boule ou tête fémorale interchangeable et elle est caractérisée en ce que la queue se termine par une pièce mâle ou fût d'une longueur de l'ordre de 3 cm ayant une section en forme d'arbre cannelé tandis que la boule ou tête fémorale présente une cavité cylindrique de même section se prolongeant hors de la sphère de façon à former dans son ensemble une pièce femelle ayant une longueur au moins égale à celle de la pièce mâle ci-dessus.

D'autre part, la queue fémorale a de préférence une section rectangulaire de forte largeur, égale ou supérieure à 7 mm environ, ce qui correspond à un surdimensionnement assurant un meilleur blocage en rotation, une diminution du volume de ciment nécessaire et une réduction des risques de fractures.

L'invention est décrite ci-après avec référence au dessin annexé sur lequel :

- la figure 1 est une vue en élévation latérale ou de profil de la prothèse de l'invention, la boule étant séparée de la sphère et vue en coupe ;

- la figure 2 est une épure à échelle agrandie de la section de la queue de la prothèse de la figure 1 ;

- la figure 3 est une vue analogue à la

B 8275.3 AM

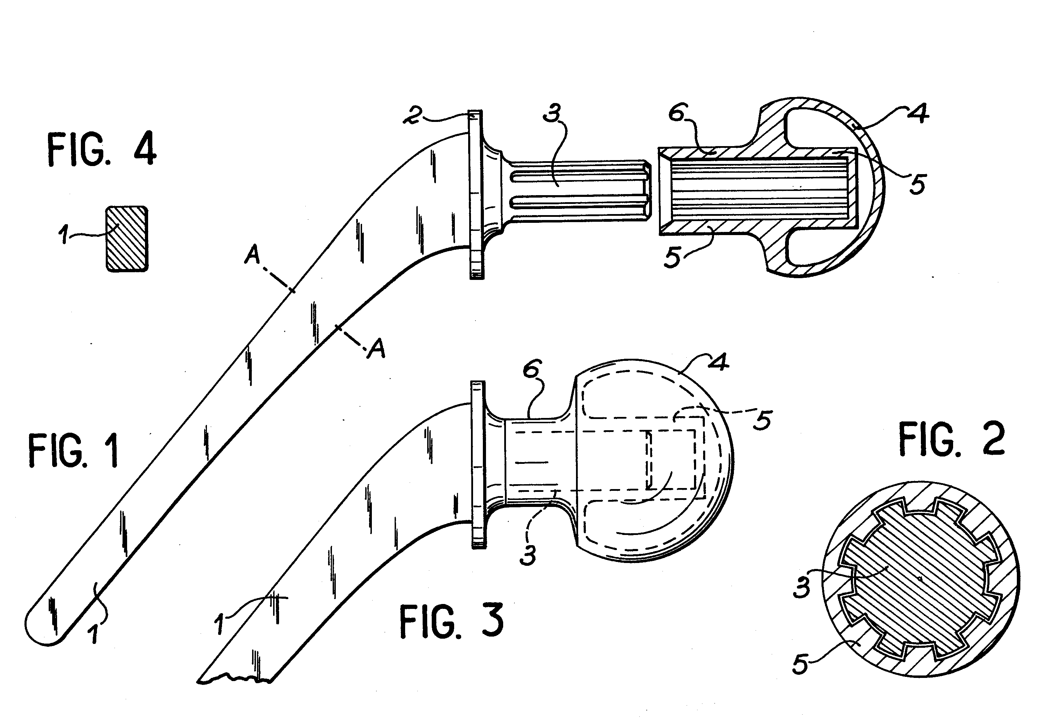

figure 1 avec une boule de diamètre différent ;

- la figure 4 est une coupe selon A-A de la figure 1.

En se reportant à ces figures, on voit que la prothèse se compose d'une queue diaphysaire 1 terminée par une collerette 2 portant une pièce mâle ou fût 3, en forme de cylindre d'une longueur relativement grande, de l'ordre de 3 cm et de section en forme d'arbre cannelé selon la figure 2 (le nombre et la profondeur des cannelures pouvant varier), combinée avec une tête fémorale 4 portant une cavité cylindrique 5 de même section que le fût 3 avec une tolérance d'emboîtement inférieure à 0,1 mm, la cavité 5 s'étendant à l'intérieur de la sphère 4 et débordant celle-ci en 6 telle que sa longueur soit supérieure à celle du fût 3.

On peut ainsi :

- dans un premier temps, poser la pièce fémorale et y adapter la tête fémorale du diamètre identique à la tête fémorale du patient que l'on a otée

- changer cette tête fémorale prothétique sans toucher à la pièce fémorale déjà scellée,

- si on le juge utile, après avoir essayé la congruence tête fémorale prothétique-cotyle, on peut choisir une taille de tête fémorale plus grande ou plus petite afin d'obtenir une congruence tête fémorale prothétique-cotyle parfaite et surtout une stabilité de l'articulation,

- surtout ce système permet de pouvoir "totaliser", longtemps après l'intervention initiale, une prothèse fémorale en place, par le simple changement de la tête fémorale en une tête de diamètre appropriée qui viendra en articulation dans un cotyle prothétique implanté au niveau de l'os iliaque.

B 8275.3 AM

4                                00124443

L'opérateur ne sera pas obligé de retirer, après descellement, ce qui est toujours préjudiciable, la queue fémorale solidaire de l'axe d'emboîtement. Il lui suffira de retirer la pièce femelle et mettre à sa place celle qui devra s'adapter au cotyle prothétique.

La section en arbre cannelé assure la transmission des efforts latéraux et de rotation tout en laissant la liberté de translation permettant une interchangeabilité efficace et elle peut être réalisée avec toute la précision souhaitable.

Cette liberté de translation a un effet d'amortissement au contact tête prothétique-cotyle humain ce qui diminue la vitesse d'usure de ce dernier.

Il est possible de prévoir un jeu de têtes fémorales 4 couvrant la variabilité anatomique, de 36 mm à 54 mm de diamètre externe environ, rencontrée chez l'homme.

La queue est de préférence de section rectangulaire à bords arrondis ayant une forte largeur, comme représenté figure 4, cette largeur étant au moins égale à 7 mm et de préférence de l'ordre de 9 mm. De telles dimensions correspondent à un surdimensionnement par rapport au cas moyen dont l'avantage est un meilleur blocage en rotation et une diminution du volume du ciment nécessaire. Les risques de fractures de la queue s'en trouvent réduits.

La prothèse selon l'invention peut être réalisée en différents matériaux, tels que l'acier ou le titane, poreux ou non, les matériaux composites, poreux ou non, les biomatériaux carbonés permettant ou non la réhabilitation osseuse à l'intérieur de la surface de certains de ces éléments prothétiques.

L'arthroplastie peut être partielle fémorale simple, le sacrifice osseux respectant le cotyle, ou

B 8275.3 AM

totale, dans le cas d'implantation d'une pièce prothétique dans ce cotyle.

Ou encore on peut réaliser une prothèse dite
intermédiaire avec une pièce fémorale et une cupule
intracotyloïdienne, mobile à la fois autour de la tête
et dans le cotyle, dont il existe de nombreux modèles.

La partie cotyloïdienne n'est pas représentée
car elle ne fait pas partie de l'invention, à laquelle
on peut adapter différents modèles existants.

B 8275.3 AM

## REVENDICATIONS

1. Prothèse fémorale de la hanche composée d'une queue (1) et d'une tête (4), caractérisée en ce que la queue se termine par une pièce mâle ou fût (3) d'une longueur de l'ordre de 3 cm ayant une section en forme d'arbre cannelé tandis que la boule ou tête fémorale (4) présente une cavité cylindrique (5) de même section se prolongeant en (6) hors de la sphère de façon à former dans son ensemble une pièce femelle ayant une longueur au moins égale à celle de la pièce mâle ci-dessus.

2. Prothèse selon la revendication 1, caractérisée en ce que la tolérance d'emboîtement de la tête (4) sur le fût (3) est inférieur à 0,1 mm.

3. Prothèse selon l'une quelconque des revendications 1 ou 2, caractérisée en ce que la queue fémorale a de préférence une section rectangulaire de forte largeur, égale ou supérieure à 7 mm environ pour les cas moyens.

FIG. 2

FIG. 3

FIG. 4

FIG. 1